# EUROPEAN PATENT APPLICATION

(11) **EP 3 819 008 A1**
(43) Date of publication of application: **12.05.2021**
(21) Application number: 20206133.9
(22) Date of filing: 06.11.2020
(51) Int. Cl.: A61Q 3/02, A61K 8/85, A61K 8/73

(54) **AN ANHYDROUS COMPOSITION FOR NAIL POLISHES AND A METHOD OF TREATING NAILS WITH THE POLISH COMPOSITION**

(30) Priority: 11.11.2019 IT 201900020748
(71) Applicant: Chromavis S.p.A., 20122 Milano (IT)
(72) Inventor: Fularska, Anna, 06-300 Przasnysz (PL); Borzym, Justyna, 09-100 Plonsk (PL); LABORDE-BERBESSON, Anne, 24400 MUSSIDAN (FR); OFFENSTEIN, Caroline, 33290 BLANQUEFORT (FR)
(74) Representative: Vanosi, Adelio Valeriano

(57) **Abstract**

An anhydrous composition for nail polishes is disclosed, as well as a method of treating or decorating nails with said composition. The polish anhydrous composition of the invention gives remarkable effects on nails, either decorative or functional to decoration, while at the same time encompassing naturally-based ingredients allowing the exclusion of allergenic polymers.

## Description

### FIELD OF THE INVENTION

The present invention relates to anhydrous compositions for nail polishes, and to a method of treating nails with said compositions.

### STATE OF THE ART

The prior art for decorating fingernails provides for the application of a layer of coloured, transparent, pearly, etc. nail polish on the nails using a suitable brush.

In order to obtain special effects, after applying the coloured base polish, artistic, geometric or pattern designs are often drawn on the polish using a thin brush to revive the polish with pearlescent, iridescent or glittered visual effects by applying a further polish layer which generates such effects.

Nail polishes are also known which provide for the application of a transparent base polish layer which reinforces and regenerates the nail, and then a coloured layer which gives the desired colour to the nail, on top of which a top coat may be added, which acts as a fixer, shining aid and staying power enhancer.

With changing fashion, consumers, who are increasingly demanding, are looking for novel make-up products which confer original or specific make-up effects. A need therefore remains to have available a polish which, when applied to nails, results in a make-up effect different from those of the smooth, continuous and homogeneous films typically obtained with the commercially available products.

At the same time, however, nail polishes can have toxic chemicals that are harmful for both the users and the environment. Therefore, it is desirable to improve also the overall sustainability and eco-friendly nature of these beauty products.

In this regard, it is the object of the present invention to provide a polish for nails, which is highly performant while involving more natural or naturally-based components, so as to result in a more environmentally-friendly formulation.

### SUMMARY OF THE INVENTION

This and other objects are achieved by the use of capryloyl glycerine/sebacic acid copolymer as a film-forming agent in anhydrous composition for nail polishes.

In another aspect, the present invention relates to an anhydrous composition for nail polishes and by a method of treating or decorating them by means of said composition, implemented according to the technical teachings of the appended claims.

In a further aspect, the present invention relates to the use of the anhydrous composition as a nail base coat polish and as a nail top coat polish.

The characteristics and the advantages of the present invention will become apparent from the following detailed description, from the working examples provided for illustrative purposes, and from the accompanying Figure, wherein:

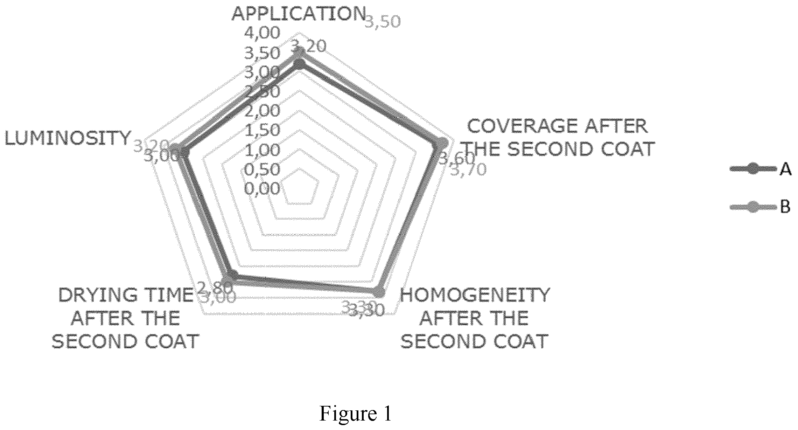

Figure 1 shows the comparative results obtained in Example 5.

### DETAILED DESCRIPTION OF THE INVENTION

The anhydrous composition for nail polishes of the present invention comprises at least a plasticizer, at least a solvent, at least a rheological agent, at least a colouring agent, and at least a film forming agent comprising a capryloyl glycerine/sebacic acid copolymer.

Said capryloyl glycerine/sebacic acid copolymer is an ester formed by the reaction of glycerine, sebacic acid, and caprylic acid. Advantageously, all these starting materials are derived from vegetable sources only.

It has been surprisingly found that the presence of this copolymer in the polish anhydrous composition allows not only to increase the overall sustainability and eco-friendly nature of the final nail polish, but also to avoid the use of acrylates or methacrylates, which are typically used as film forming agents in polish compositions. In this regard, acrylates or methacrylates are known to cause allergic reaction. In a study carried out in UK and Ireland in 2017, most patients developed their allergy through using nail enhancements, nail or eyelash glue - and a third were affected working as nail beauticians.

Additionally, acrylates and methacrylates are not readily biodegradable, especially with respect to polyester resins.

Therefore, it is greatly appreciable that the polish anhydrous composition of the invention gives remarkable effects on nails, either decorative or functional to decoration, while at the same time encompassing naturally-based ingredients allowing the exclusion of allergenic polymers. Thus, preferably, the polish anhydrous composition of the invention does not comprise acrylates and methacrylates.

The capryloyl glycerine/sebacic acid copolymer allows to give unexpected great results on nails also because it has been surprisingly found that said copolymer offers very good adhesion properties. The copolymer of the invention has been compared with a synthetic polyester-polyacrylic resin and with a citric acid/propylene glycol/succinic acid copolymer. The results are reported in the table below:

| **INCI name of resin** | Adipic Acid/Neopentyl Glycol/ Trimellitic Anhydride Copolymer, Acrylates Copolymer | Citric Acid/Propylene Glycol/Succinic Acid Copolymer | Capryloyl glycerine/sebacic acid copolymer |
|---|---|---|---|
| **Brookfield** | 3440 | 3220 | 2560 |
| **Viscosity** | 1056 | 966 | 850 |
| **RVT, sp3, 5/50/5 (cps)** | 2200 | 2780 | 1580 |
| **Ti** | **Ti=2,08** | **Ti=2,88** | **Ti=1,86** |
| **Syneresis Level - sample was kept in 1 month in oven 50°C** | Moderate | High Level, visible | No syneresis |
| **Cross Hatch Cut Test*** | 1 | 3 | 0 |

The Cross Hatch Cut Test* Method is widely used to assess the adhesion of paint coatings and provides an instant assessment of the quality of the bond to the substrate. Classification is made based on the table below:

| **Cuts' appearance** | **description** | **Classification** |
|---|---|---|
| | Completely smooth blades of the cuts, without any loss of coating material. | 0 |
| 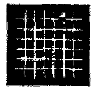 | Loss of small coating material's particles, detached from the cuts' intersections. The loss of coating material is just a little bit more than 5% of the complete Cross Hatch Cut's area. | 1 |
| 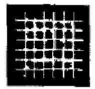 | Loss of small coating material's flakes along the cuts' blades and/or at their intersections. The loss of coating material is distinctly greater than 5% up to just a little bit more than 15% of the complete Cross Hatch Cut's area. | 2 |
| 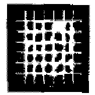 | Loss of coating material's flakes along the cuts' blades and/or of squares (partly or wholly), The loss of coating material is distinctly greater than 15% up to just a little bit more than 35% of the complete Cross Hatch Cut's area. | 3 |
| 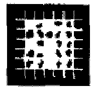 | Loss of coating material's flakes along the cuts' blades and/or of squares (partly or wholly). The loss of coating material is distinctly greater than 35% up to just a little bit more than 65% of the complete Cross Hatch curs area. | 4 |
| | Coating material's loss of distinctly more than 65%, which cannot even be classified by classification "4". | 5 |

It can be easily noted that the best product stability is obtained with capryloyl glycerine/sebacic acid copolymer. In addition, the formulation with capryloyl glycerine/sebacic acid copolymer gives much better results in adhesion tests, where it belongs to the classification 0.

Preferably, in the anhydrous composition for nail polishes of the invention, said capryloyl glycerine/sebacic acid copolymer is in an amount up to 20wt% on the film forming agent weight.

More preferably, in the anhydrous composition for nail polishes of the invention, said capryloyl glycerine/sebacic acid copolymer is in an amount up to 15wt% on the film forming agent weight.

In particularly preferred embodiments, in the anhydrous composition for nail polishes of the invention, said capryloyl glycerine/sebacic acid copolymer is in an amount of 2-12wt% on the film forming agent weight.

The anhydrous composition of the invention can be a nitrocellulose-based nail polish, a water-based nail polish or a sprayable nail polish.

Preferably, the anhydrous composition of the invention is a nitrocellulose-based nail polish, because the film forming agent comprises nitrocellulose (cellulose nitrate - cellulose origin can be cotton linters and/or wood pulp). Nitrocellulose can be purchased in various viscosities to match the desired viscosity of the final product.

Preferably, nitrocellulose is a nitrocellulose of RS types with 11,5-12,4% nitrogen content (other types can be used for example -RS ½ second, -RS ¼ second, - RS 5/6 second), or a mixture thereof.

In preferred embodiments, the film forming agent comprises nitrocellulose in an amount up to 90wt% on the film forming agent weight.

In more preferred embodiments, the film forming agent of the composition of the invention nitrocellulose and capryloyl glycerine/sebacic acid copolymer are in a weight ratio of 2:1 to 25:1, more preferably of 5:1 to 15:1.

In the most preferred embodiments, the film forming agent consists of nitrocellulose and capryloyl glycerine/sebacic acid copolymer.

In some embodiments, said capryloyl glycerine/sebacic acid copolymer is in an amount up to 10wt% on the anhydrous composition weight.

More preferably, said capryloyl glycerine/sebacic acid copolymer is in an amount up to 5wt% on the anhydrous composition weight.

In particularly preferred embodiments, said capryloyl glycerine/sebacic acid copolymer is in an amount of 0.5-3wt% on the anhydrous composition weight.

The anhydrous composition of the invention also contains at least a plasticizer, at least a solvent, at least a rheological agent, and at least a colouring agent, whereas, as being anhydrous, it contains no water.

Preferably, the anhydrous composition of the invention comprises 5-25wt% of at least a film forming agent, on the weight of the anhydrous composition, more preferably 10-25wt%.

In addition to said capryloyl glycerine/sebacic acid copolymer, and nitrocellulose, other suitable film-forming agents are:
- cellulose film-forming derivatives, preferably cellulosic esters, such as cellulose acetate, cellulose acetate butyrate, cellulose acetate propionate, cellulose ethers or a mixture thereof,
- synthetic polymers, such as polyesters, epoxy resins, vinyl resins, poly(vinyl alcohol), crosslinked poly(vinyl alcohol), polybutene, VP/VA copolymer, polyurethane, hydrogenated polycyclopentadiene, or a mixture thereof,
- silicone resins, preferably trimethylsiloxysilicate, polymethylsilsesquioxane, C26-28 Alkyl Dimethicone,
- ether-based resins, preferably polyvinyl stearyl ether.

Plasticizers are added to improve flexibility and mechanical resistance of the final polish film on nails. Preferably, the anhydrous composition of the invention comprises 1-10wt% of at least a plasticizer, on the weight of the anhydrous composition, more preferably 4-10wt%.

Suitable plasticizers are castor oil, amyl and butyl stearate, citrates (such as acetyl triethyl citrate, acetyl tributyl citrate, acetyl trihexyl citrate, triethyl citrate), adipates, sebacates, sucrose acetate isobutyrate, sucrose benzoate, glycol esters, fatty acids, camphor, 2,2,4-trimethyl-1,3-pentanediol diisobutyrate, dipropylene glycol dibenzoate, and trimethylpentanediyl dibenzoate.

Preferred plasticizers are citrates selected from acetyl triethyl citrate, acetyl tributyl citrate, acetyl trihexyl citrate, triethyl citrate, and mixtures thereof.

The anhydrous composition also contains at least a rheological agent, which may act to thicken the composition in order to suspend the colouring agents (such as mainly pigments and pearls), while saving a better spreading on the nail.

Preferably, the anhydrous composition of the invention comprises up to 5wt% of at least a rheological agent, on the weight of the anhydrous composition, more preferably up to 2wt%.

The typical rheological agents used in nail polish compositions are organically-treated bentonite and hectorite. Preferably, said at least a rheological agent is selected from stearalkonium hectorite, stearalkonium bentonite, montmorillonite clays, treated clays, and mixtures thereof.

Other suitable rheological agents are silica, and thixotropic alkyds.

The colouring agents and other components of nail polish are typically contained within one or more solvents, until the polish is applied. After application, the solvent must be able to evaporate.

Preferably, the anhydrous composition of the invention comprises 50-70wt% of at least a solvent, on the weight of the anhydrous composition.

Butyl acetate, isobutyl acetate, ethyl acetate, propyl acetate, isopropyl acetate, acetone, ethyl alcohol, and isopropyl alcohol are the most commonly used solvents, but silicone-based solvents such as silanes and siloxanes are also suitable. Other suitable solvents are amyl acetate, heptane, 1-butanol, 2-butyl alcohol, methyl ethyl ketone, and water.

Preferably, the anhydrous composition of the invention comprises up to 10wt% of at least a colouring agent, on the weight of the anhydrous composition. Choice of colouring agent (or pigment) and its ability to be well dispersed and properly suspended is essential for producing a good quality product.

In addition to usual colouring agents, other colour tones can be added depending upon the colour, tone, and hue of the desired product. Natural and synthetic mica-based products, borosilicates, bismuth oxychloride called 'pearl essence' are white, iridescent or coloured common additives, that can be mixed also with gold, silver, and bronze tones.

Optionally, the anhydrous composition may further comprise at least one cosmetically acceptable additive selected from surfactants, emulsifiers, emollients, moisturizers, humectants, fragrances, biocides, preservatives, chelating agents, antioxidants, antimicrobial agents, anti-fungal agents, antiperspirant agents, exfoliants, vitamins, alpha-hydroxy acids, beta-hydroxy acids, retinols, niacinamide, lightening agents, absorbing agents for ultraviolet radiation, botanical extracts, organic oils, waxes, high refractive index materials, and mixtures thereof.

In preferred embodiments, the anhydrous composition comprises:
5-25wt% of at least a film forming agent,
1-10wt% of at least a plasticizer,
50-70wt% of at least a solvent,
up to 5wt% of at least a rheological agent, and
up to 10wt% of at least a colouring agent,
on the weight of the anhydrous composition.

More preferably, the anhydrous composition comprises:
10-25wt% of at least a film forming agent,
4-10wt% of at least a plasticizer,
50-70wt% of at least a solvent,
up to 2wt% of at least a rheological agent, and
up to 10wt% of at least a colouring agent,
on the weight of the anhydrous composition.

In the most preferred embodiments, the anhydrous composition comprises:
10-25wt% of at least a film forming agent comprising nitrocellulose and capryloyl glycerine/sebacic acid copolymer,
4-10wt% of at least a plasticizer,
50-70wt% of at least a solvent,
up to 2wt% of at least a rheological agent, and
up to 10wt% of at least a colouring agent,
on the weight of the anhydrous composition.

The anhydrous composition for nail polishes as above described is in a liquid form at room temperature and can be applicable directly on nails, for example by using a brush

In some embodiments, the anhydrous composition for nail polishes of the present invention consists essentially of at least a plasticizer, at least a solvent, at least a rheological agent, at least a colouring agent, and at least a film forming agent comprising a capryloyl glycerine/sebacic acid copolymer, as above described. The expression "consists essentially of" means that said capryloyl glycerine/sebacic acid copolymer is the only active agent in the composition for avoiding the use of acrylates or methacrylates as film forming agents in the composition.

In other embodiments, the anhydrous composition for nail polishes of the present invention consists of at least a plasticizer, at least a solvent, at least a rheological agent, at least a colouring agent, and at least a film forming agent comprising a capryloyl glycerine/sebacic acid copolymer, as above described.

In a further aspect, the present invention relates to the use of the anhydrous composition for treating nails.

As it will be clear from the following working Examples, the composition of the invention can be used directly on nails or can be used together with other nails polishes.

When used directly on nails, the anhydrous composition of the invention allows to obtain original and specific make-up effects on nails, without causing allergic reactions on users.

In a further aspect, the present invention relates to the use of the anhydrous composition as a nail base coat polish.

In another aspect, the present invention relates to the use of the anhydrous composition as a nail top coat polish.

It should be understood that all the aspects identified as preferred and advantageous for the anhydrous composition are to be deemed as similarly preferred and advantageous also for the uses, and methods of application of the same.

It should be also understood that all the combinations of preferred aspects of the anhydrous composition of the invention, as well as of uses, and methods of application, as above reported, are to be deemed as hereby disclosed.

Below are working examples of the present invention provided for illustrative purposes.

### EXAMPLES

### Example 1.

The following composition was prepared:

| Ingredients | wt% |
|---|---|
| Film forming agent | 18.0 |
| [-Nitrocellulose (70%) and - capryloyl glycerine/sebacic acid | [16.0 2.0] |

| copolymer] | |
|---|---|
| Solvent (ethyl acetate and butyl acetate) | 67.9 |
| Acetyl tributyl citrate | 4.5 |
| Acetyl triethyl citrate | 2.0 |
| Stearalkonium bentonite | 0. 6 |
| Colouring agents (Red) | 7.0 |
| TOTAL | 100 |

### Example 2.

The following composition was prepared:

| Ingredients | wt% |
|---|---|
| Film forming agent | 18.0 |
| [-Nitrocellulose (70%) and - capryloyl glycerine/sebacic acid copolymer] | [16.0 2.0] |
| Solvent (ethyl acetate, butyl acetate) | 67.9 |
| Acetyl tributyl citrate | 6.5 |
| Stearalkonium hectorite | 0.6 |
| Colouring agents (White) | 7.0 |
| TOTAL | 100 |

### Example 3.

The following composition was prepared:

| Ingredients | wt% |
|---|---|
| Film forming agent | 18.0 |
| [-Nitrocellulose (70%) and - capryloyl glycerine/sebacic acid copolymer] | [12.0 2.0] |
| - and/or synthetic polyester resin, and /or tosylamide epoxy resin | 4.0 |
| Solvent (ethyl acetate, butyl acetate) | 67.9 |
| Acetyl tributyl citrate | 6.5 |
| Stearalkonium hectorite | 0. 6 |
| Colouring agents (White) | 7.0 |
| TOTAL | 100 |

### Example 4.

The following composition was prepared:

| Ingredients | wt% |
|---|---|
| Film forming agent | 18.0 |
| [- CAB (cellulose acetate butyrate and nitrocellulose and - capryloyl glycerine/sebacic acid copolymer] | [12.0 2.0] |
| - and/or synthetic polyester resin | 4.0 |
| Solvent (ethyl acetate, butyl acetate, isopropanol) | 75.4 |
| Acetyl tributyl citrate | 6.5 |
| Light stabilizer | 0.1 |
| TOTAL | 100 |

### Example 5.

All the compositions prepared in the previous Examples have been tested on hand nails of 20 volunteers. The compositions were applied with a brush directly on nails.

The resulting film on nails was satisfactory in terms of brightness, uniformity, colour, texture, highly tolerable to nail surface and surrounding skin.

In addition, a comparison panel test was performed on 20 people:
- sample A containing acrylic compound,
- sample B containing capryloyl glycerine/sebacic acid copolymer.

The latter resulted in better rates in almost all tested aspects: application, luminosity, drying time, coverage. Only homogeneity after one coat were similar for A and B. The graph reported in Figure 1 presents results of performed panel test.

It was also appreciated that most of the used ingredients are naturally-based, thus boosting the overall amount of the latter up to 87-89wt% on the composition weight.

## Claims

1. A nail polish anhydrous composition comprising at least a plasticizer, at least a solvent, at least a rheological agent, at least a colouring agent, and at least a film forming agent comprising a capryloyl glycerine/sebacic acid copolymer, wherein said capryloyl glycerine/sebacic acid copolymer is in an amount up to 20wt% on the film forming agent weight.

2. The anhydrous composition of claim 1, wherein said capryloyl glycerine/sebacic acid copolymer is in an amount up to 15wt% on the film forming agent weight.

3. The anhydrous composition of claim 3, wherein said capryloyl glycerine/sebacic acid copolymer is in an amount of 2-12wt% on the film forming agent weight.

4. The anhydrous composition of any one of claims 1-3, wherein the film forming agent comprises nitrocellulose in an amount up to 90wt% on the film forming agent weight.

5. The anhydrous composition of claim 4, wherein, in the film forming agent, nitrocellulose and capryloyl glycerine/sebacic acid copolymer are in a weight ratio of 2:1 to 25:1, preferably 5:1 to 15:1.

6. The anhydrous composition of claim 4 or 5, wherein the film forming agent consists of nitrocellulose and capryloyl glycerine/sebacic acid copolymer.

7. The anhydrous composition of any one of claims 1-6, wherein capryloyl glycerine/sebacic acid copolymer is in an amount up to 10wt% on the anhydrous composition weight, preferably in an amount up to 5wt%, more preferably in an amount of 0.5-3wt%.

8. The anhydrous composition of any one of claims 1-7, comprising:
5-25wt% of at least a film forming agent,
1-10wt% of at least a plasticizer,
50-70wt% of at least a solvent,
up to 5wt% of at least a rheological agent, and
up to 10wt% of at least a colouring agent,
on the weight of the anhydrous composition.

9. The anhydrous composition of claim 8, comprising:
10-25wt% of at least a film forming agent,
4-10wt% of at least a plasticizer,
50-70wt% of at least a solvent,
up to 2wt% of at least a rheological agent, and
up to 10wt% of at least a colouring agent,
on the weight of the anhydrous composition.

10. Use of the anhydrous composition of any one of claims 1-9 for treating or decorating nails.
